# EUROPEAN PATENT SPECIFICATION

(11) **EP 4 674 455 B1**
(45) Date of publication and mention of the grant of the patent: **09.12.2026**
(21) Application number: 24186195.4
(22) Date of filing: 03.07.2024
(51) Int. Cl.: A61M 16/06

(54) **FOREHEAD PAD FOR RESPIRATORY MASK**
STIRNPOLSTER FÜR ATEMMASKE
COUSSINET FRONTAL POUR MASQUE RESPIRATOIRE

(43) Date of publication of application: 07.01.2026
(73) Proprietor: Air Liquide Medical Systems, 92182 Antony Cedex (FR)
(72) Inventor: ALBERICI, Luca, 25073 Bovezzo (IT); ROMAIOLI, Roberto, 25073 Bovezzo (IT); RUOCCO, Alessandra, 25073 Bovezzo (IT)
(74) Representative: Air Liquide

(56) References cited:
- EP-A1- 2 668 971
- WO-A2-03/082406
- CN-U- 217 697 577
- DE-A1- 102016 013 437
- US-A1- 2021 170 130
- US-B2- 7 735 487

## Description

The present disclosure concerns a forehead pad for a respiratory mask, typically a facial mask, and a respiratory mask comprising such a forehead pad that is useable in the treatment of respiratory conditions or diseases affecting adults, children, toddlers, infants or newborns.

Respiratory masks are commonly used for delivering a flow of breathable or respiratory gas, such as air under pressure, to a person, i.e. a patient, for treating respiratory various conditions or diseases affecting that person, for instance obstructive sleep apnea (OSA). Masks receiving only the patient's nose are called a nasal mask, whereas those receiving the patient's nose and mouth are called facial or oro-nasal masks.

A mask assembly typically comprises a rigid or semi-rigid mask body and a soft face-contacting cushion, commonly called a flexible cushion, that comes into contact with the patient's face and conforms to the various facial contours of the patient face for receiving the patient's nose or nose and mouth so that the patient can inhale the gas. The flexible cushion is usually made of soft, resilient elastomeric material, such as soft silicone or similar material.

Some masks further comprise an upper arm that projects upwardly and carries a forehead pad or cushion that comes into contact with the forehead of the patient, when donned by the patient. The upper arm often comprises headgear connecting structures for fixing a headgear thereto, for pulling the mask against the face with sufficient force to achieve a gas tight seal between the mask and the wearer's face, and to further maintain the mask in position on the patient's face, while used, i.e. during their treatment.

Various examples of respiratory mask assemblies are disclosed by EP-A-462701, EP-A-874667, EP-A-1972357, EP-A-3981455, WO-A-00/57942, EP-A-2708258 and US-A-2007/0044804.

Various examples of forehead pads for respiratory masks are disclosed by WO 03/082406 A2, EP 2 668 971 A1, US 7 735 487 B2, DE 10 2016 013437 A1, CN 217 697 577 U and US 2021/170130 A1.

Despite the fact that many different architectures of masks have been proposed, some problems or drawbacks still exist, especially related to the discomfort of use for the patient. Indeed, various causes of discomfort exist, such as gas leaks due to a wrong assembling of the mask components, a poor positioning and/or stability of the mask of the patient's face, loose settings of the headgear, poor tightness of the cushion...

The forehead pad can be a cause of discomfort for some patients. Indeed, as it comes into direct contact with the forehead of the patients, when the mask is donned, the forehead pad can hurt the patient in their forehead regions, for instance can cause some irritations, "red marks" or similar effects on said forehead regions, especially when the headgear pulls the mask against the face with a strong force to achieve an efficient gas tight sealing and good maintaining in position.

To overcome such discomfort, it has been proposed to use some forehead pads comprising a hollow body made of a resilient material that can be deformed or crushed, while the headgear is pulling the mask against the face. An example of such a forehead pad is taught by EP-A-2329858. However, such forehead pads are not ideal as they can create other issues, such as a lateral "sliding" of the pad structure leading to instabilities in the positioning and maintaining of the mask on the patient's face.

In this context, a problem is to improve the forehead pad of a respiratory mask, especially a facial mask, for limiting or avoiding the "sliding" phenomena and for thereby improving the positioning and maintaining of the mask on the patient's face.

### Summary of the invention

The present invention provides a forehead pad for a respiratory mask as defined in claim 1. The present invention also provides a respiratory mask comprising such a forehead pad as defined in claim 14. Further preferred embodiments of the present invention are defined in the dependent claims.

### Summary of the disclosure

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

The present disclosure concerns a forehead pad for a respiratory mask, especially a facial mask, comprising:
- a hollow main body having an elongated tubular shape and comprising an inner volume, i.e. a hollow lumen, and
- a fixation structure secured to an outer wall of the hollow main body and configured for being attachable to the respiratory mask.

Further, the forehead pad comprises a plurality of reinforcing members arranged in the inner volume of the hollow main body, each reinforcing member comprising a first edge secured to an inner wall of the hollow main body and a second edge secured to at least another reinforcing member.

Furthermore, at least the hollow main body and the reinforcing members are made of a resilient material, i.e. flexible or deformable.

The forehead pad according to the present disclosure can further comprise one or more of the following features :
- the reinforcing members have an elongated shape, i.e. they are elongated, so as to constitute elongated reinforcing members.
- the elongated reinforcing members are axially-arranged in the hollow main body.
- each elongated reinforcing member comprises a first longitudinal edge secured to the inner wall of the hollow main body and a second longitudinal edge secured to at least one of the other elongated reinforcing members, preferably to (all) the other elongated reinforcing members.
- the forehead pad comprises at least 4 reinforcing members, preferably 4 reinforcing members attached together.
- the second edges of the reinforcing members are secured together in a common junction region, especially the second longitudinal edges of the elongated reinforcing members.
- the junction region is located in a center region of the inner volume of the hollow main body.
- the elongated reinforcing members are substantially radially-arranged in the inner volume of the hollow main body.
- the elongated members have a blade shape, a plate shape or the like.
- the elongated members are (slightly) curved.
- the 4 reinforcing members are attached together along their second edges so as to form a substantially "X"- shape structure, in cross-sectional view.
- the 4 elongated members are attached together along their second longitudinal edges so as to form a substantially "X"- shape structure, in cross-sectional view.
- preferably the substantially "X"- shape structure is a curved-arm "X"- shape structure.
- the fixation structure comprises an elongated base part and an elongated neck part secured together, the neck part linking the base part to the outer wall of the hollow main body.
- the base part of the fixation structure has a base width W₁ that is larger than a neck width W₂ of the neck part, i.e. W₁ > W₂.
- the base width W₁ is of between 7 and 17 mm, preferably between 9 and 13 mm, for instance about 11 mm.
- the neck width W₂ is of between 2 and 8 mm, preferably of between 2.5 and 6.5 mm, for instance from about 3 mm in the thinnest region of the bottom to about 5.5 mm in the thickest region of the top.
- the base part forms with the neck part, a substantially "T"-shape structure, in cross-sectional view.
- the base part has a plate or blade shape, or the like.
- the hollow main body, the reinforcement members and the fixation structure are made one piece of resilient material, preferably by extrusion or the like.
- the resilient material is chosen among silicone materials, e.g. silicones adapted to extrusion processes.
- the length of the elongated reinforcing members is substantially equal to the length of the inner volume of the hollow main body.
- the hollow main body has a length (L) of between 35 and 70 mm, preferably of between 45 and 60 mm, for instance of about 50 mm.
- the hollow main body has a thickness (T) (or diameter) of between 15 and 25 mm, preferably of between 17 and 21 mm, for instance of about 19 mm.

The present disclosure also concerns a respiratory mask, preferably a facial mask, comprising a mask body comprising an upper arm projecting upwardly; forehead pad coupling structures arranged at a free end of the upper arm; and a forehead pad according to the present disclosure, e.g. as above described, secured, i.e. attached, to the forehead pad coupling structures.

The mask according to the present disclosure can further comprise one or more of the following features :
- the forehead pad coupling structures comprise two guiding rails that are parallel to each other, for receiving and securing the forehead pad.
- the two guiding rails cooperate with the base part of the fixation structure of the forehead pad.
- the forehead pad coupling structures further comprise an abutment for stopping the course of the forehead pad, during its insertion between the rails.

Further, depending on the embodiment, the respiratory nasal mask of the disclosure can also comprise one or more of the following additional features :
- it comprises a flexible cushion.
- the flexible cushion is detachably secured to the main body.
- the main body comprises a front opening.
- the main body comprises two lateral pockets or lodging for receiving and securing headgear connectors.
- alternatively, the main body comprises two lateral arms projecting laterally on each side of said mask body, i.e. on the left and right sides of the mask body, and in opposite directions.
- the upper arm is made one piece with the main body.
- the upper arm and the main body are made of polymer material, preferably a rigid or semi-rigid polymer material, such as polycarbonate (PC), polypropylene (PP), polybutylenepterephtalate (PBT), Nylon^{®}, or the like.
- the main body is made of a transparent plastic material, preferably the upper arm and the main body are made of a transparent plastic material.
- the main body defines an inner chamber, compartment or volume that contains the respiratory gas to be administered to the user, i.e. patient
- the main body has a generally-trapezoidal or triangular shape.
- the upper arm further comprises headgear connecting structures, such as slots, holes, hooks or the like.
- the cushion is sized and designed for covering the regions of the nose and of the mouth of the user, i.e. it is a facial mask.
- the cushion is made of a flexible material, i.e. a supple and soft or semi-soft material, such as silicone or the like.
- the cushion comprises a rear opening that is configured to receive the nose and mouth regions of the user, i.e. the patient, when the mask is donned.
- the peripheral edge regions surrounding the rear opening of the flexible cushion comprise a peripheral thin wall, such as a membrane or the like, constituting a sealing, preferably a supple sealing skirt, so as to ensure an efficient gas tightness while in contact with the user's face, when the latter wears the mask, preferably all along the outer periphery of said rear opening.
- the main body and the cushion comprise connecting structures for attaching the cushion directly to the mask body, in a detachable manner so that the user can disassemble them for cleaning operations for instance.
- a headgear is attached to the mask.
- the headgear comprises straps that are used for positioning, maintaining and/or securing the mask in a desired position on the facial regions of the patient, preferably straps made of polymer or fabric material, or both.
- the headgear constitute a tridimensional flexible structure comprising elongated flexible bands, ribbons or the like.
- the straps comprise Velcro^{®}-like portions for forming loops or the like and allowing attaching the headgear to the headgear connecting structures of the mask.

Furthermore, a flexible hose can be fluidly connected to the front opening of the mask body of the mask according to the present disclosure, by means of a hollow connector arranged at a free end of said flexible hose, such as an elbow shape connector. Preferably, the elbow connector is detachably fixed to the main body of the mask and rotatable with respect to the main body. The flexible hose conveys the respiratory gas that is delivered by a gas source, such as a medical ventilator or the like.

The disclosure also concerns a respiratory assembly or installation for providing gas to a patient (P) comprising a gas delivery device, such as a medical ventilator, i.e. a respiratory assistance device or the like, and a mask according to the present disclosure, wherein the gas delivery device is connected to the mask by means of the flexible hose.

The respiratory assembly and/or the mask according to the present disclosure are usable in the treatment of respiratory conditions or diseases affecting adults, children, toddlers, infants or newborns, such as obstructive sleep apnea (OSA), Obstructive Sleep Apnea Syndrome (OSAS), Obesity Hypoventilation Syndrome (OHS), Neuromuscular disease (NMD) or the like.

Non-limitative embodiments of a respiratory mask and of a forehead pad according to the present disclosure are shown in the enclosed Figures, among which:
- Figure 1 is an exploded view of a facial mask according to one embodiment of the present disclosure.
- Figure 2 shows an embodiment of the forehead pad according to the present disclosure that is arranged on the facial mask of Fig. 1.
- Figure 3 is a cross-sectional view of the forehead pad of Fig. 2.
- Figure 4 & 5 illustrate the forehead pad of Fig. 2 in use.
- Figure 6 illsutrates the coupling of the forehead pad of Fig. 2 to the mask of Fig. 1.
- Figure 7 shows the mask of Fig. 1, assembled and equipped with a headgear.

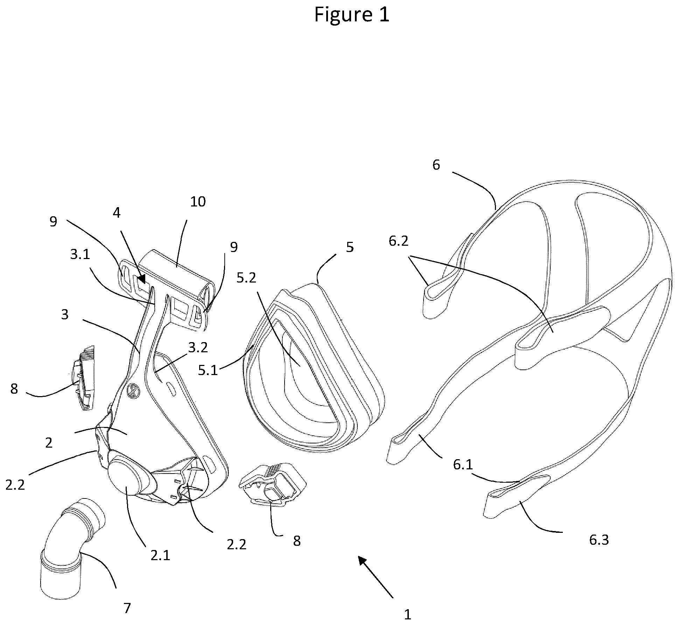

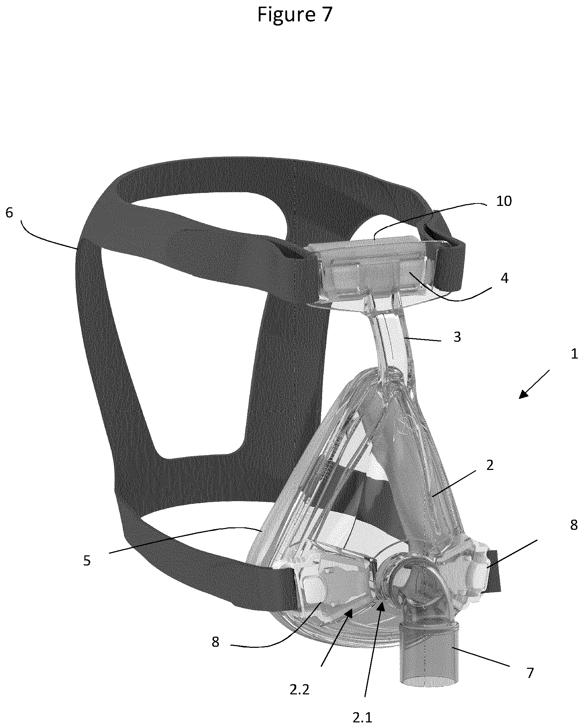

The present disclosure proposes a respiratory mask 1, preferably a facial mask covering the nose and mouth regions of a user, i.e. a patient, as shown in the Figures that illustrate an embodiment of a facial mask 1 according to the present disclosure. The mask 1 is shown in Fig. 1 & 7.

The mask 1 comprises a main body 2 and a flexible cushion 5 attached together in a detachable manner so that they can be disassembled for instance for cleaning operations or for replacing a worn out cushion 5, and easily reassembled afterwards. The flexible cushion 5 is a tridimensional hollow flexible structure made of a resilient material, such as silicone or the like, whereas the main body 2 can be made of (semi-)rigid plastic material or the like, such polypropylene PP, polycarbonate PC, polybutylenepterephtalate PBT, Nylon^{®}, or the like. Preferably, the main body 2 is made of a transparent plastic material.

The main body 2 defines an inner chamber that contains a respiratory gas to be provided to the user, i.e. patient. The main body 2 has a generally-trapezoidal or triangular shape as shown in Fig. 1&7.

The respiratory gas, such as air, is provided by a flexible hose (not shown) fluidly and mechanically connected to the main body 2, i.e. mask body, by means of a tubular elbow connector 7 arranged an end of the hose that is plugged in the central opening 2.1 of the main body 2 for providing the gas. The other end of the flexible hose comprises another connector for fluidly and mechanically connecting the hose to a medical ventilator (not shown) or a similar apparatus that provides the respiratory gas.

The mask main body 2 further comprises two lateral pockets or lodgings 2.2 arranged on each side of the main body 2, i.e. one on the right side and the other on the left side of the main body 2. Those pockets 2.2 are dimensioned and configured for lodging and holding headgear connectors 8 that are used for fixing the headgear 6 thereto, in particular the two flexible bottom straps 6.1 of the headgear 6 as shown in Fig. 1.

The headgear connectors 8 comprise headgear connecting structures, such slots, holes, hooks or the like, for attaching a headgear thereto, typically the bottom straps 6.1 of a headgear 6.

The headgear 6 is used for maintaining and securing the mask 1 in a desired position on the head/face of the patient.

In another embodiment (not shown), the main body 2 comprises two lateral arms in lieu of the pockets 2.2, namely a right and a left arm projecting laterally toward the right and left cheeks of the patient, when the mask is donned, each comprising a free end also equipped with headgear connecting structures.

The flexible cushion 5 comprises fastening means 5.1, such as wall structures or the like, cooperating with other fastening means, such as wall structures or the like, arranged on the main body 2 for allowing the attachment of the cushion 4 to the main body 2.

The cushion 5 further comprises a rear breathing opening 5.2 that receives the nose and mouth regions of the patient thereby allowing, in use, gas exchanges with the airways of the patient. The breathing opening 5.2 can comprise peripheral supple edge regions, such as a flexible membrane, surrounding said rear breathing opening 5.2 that, in use, come into contact with patient's face areas, especially the nose and mouth regions in case of a facial mask, thereby ensuring a gas tight sealing.

The main body 2 and/or the elbow connector 7 can further comprise venting orifices (not shown), i.e. traversing holes, slots or similar, for venting to the atmosphere at least a part of the CO₂-containing gaseous flow expired by the user, i.e. patient, during their expiratory phases.

The main body 2 of the mask 1 further comprises an upper arm 3 projecting upwardly from the main body 2 as shown in Fig. 1. The proximal end 3.2 of the upper arm 3 is secured to the main body 2, preferably made one piece, for instance by injection molding of the like.

The distal end 3.1 of the upper arm 3, also called its free end, also comprises headgear connecting structures 9, such slots, holes, hooks or the like, for attaching the headgear 6 thereto, typically the top straps 6.2 of the headgear 6. Preferably, the top and bottom straps 6.1, 6.2 of the headgear 6 are flexible and made of polymer or fabric materials.

The straps 6.1, 6.2 can comprise Velcro^{®}-like portions 6.3 forming loops or the like for allowing attaching the headgear 6 to the headgear connecting structures 9 of the mask 1.

Furthermore, in the aim of improving the comfort of use for the patient, the free end 3.1 of the upper arm 3 is equipped with forehead pad coupling structures 4 for fixing a forehead pad 10 thereto, as shown in Fig. 1 and Fig. 4-6.

The forehead pad 10, also called a forehead cushion, is a tridimensional flexible or supple structure that contacts the patient's forehead, while the mask 1 is donned.

In the frame of the present disclosure, the structure of the forehead pad 10 has been improved for limiting the lateral "sliding" of the pad structure and improving the stability in the positioning and maintaining of the mask 1 on the patient's face.

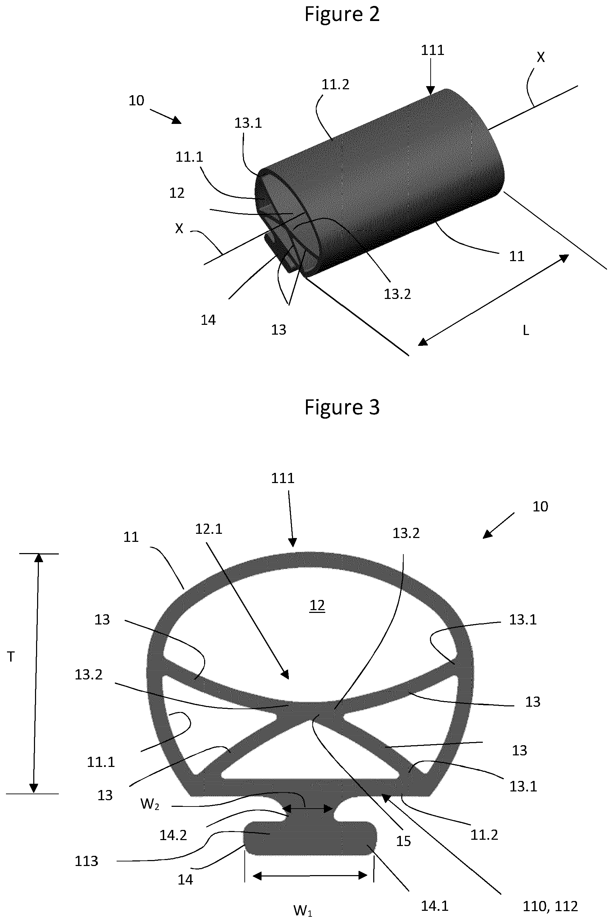

An embodiment of a forehead pad 10 according to the present disclosure is shown in Fig. 2-7.

It comprises a hollow main body 11 having an elongated tubular shape and further comprising an inner volume 12, i.e. a hollow lumen or the like. The hollow main body 11 extends along a longitudinal axis XX. The hollow main body 11 constitutes a tridimensional structure.

A fixation structure 14 is provided at the bottom part of the hollow main body 11 for attaching the forehead pad 10 to the respiratory mask 1. The fixation structure 14 is secured to an outer wall 11.2 of the hollow main body 11, in particular in an outer flat area/region 110 located under the bottom part of the main body 11 as shown in Fig. 3. The fixation structure 14 cooperates with the forehead pad coupling structures 4 of the upper arm 3 as detailed hereafter.

As shown in Fig. 2&3, the fixation structure 14 of the forehead pad 10 according to the present disclosure, comprises an elongated base part 14.1 and an elongated neck part 14.2 attached together. The neck part 14.2 links the base part 14.1 to the outer wall 11.2 of the hollow main body 11, especially to the outer flat area 110.

Preferably the base part 14.1 forms with the neck part 14.2 a substantially "T"-shape structure, when shown in cross-sectional view (see Fig. 3).

The base part 14.1 of the fixation structure 14 has a base width W₁ that is larger than a neck width W₂ of the neck part (14.2), i.e. W₁ > W₂. For instance, the base width W₁ is of about 11 mm, whereas the neck width W₂ is of between about 3 mm and about 5.5 mm.

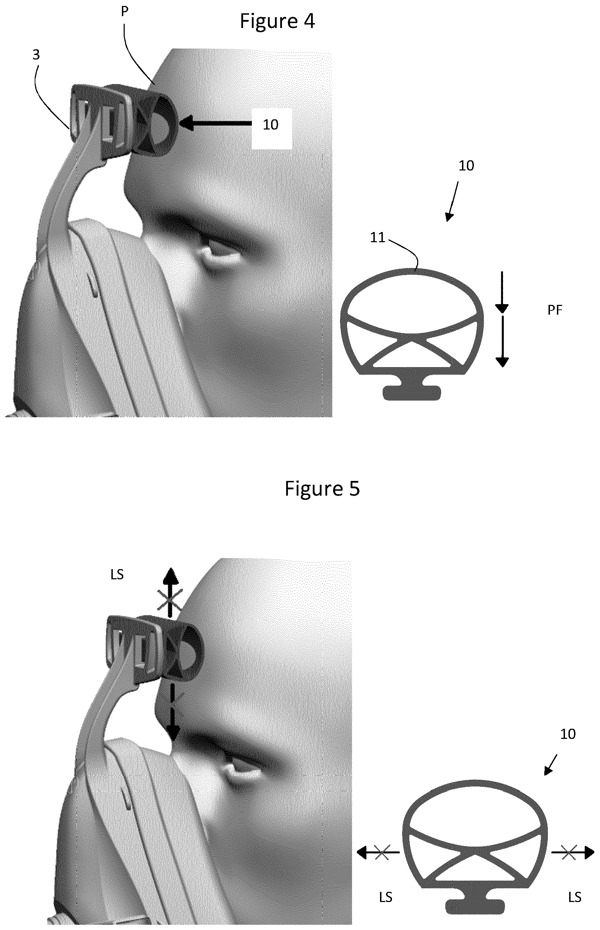

The hollow main body 11 is made of a resilient material, such as silicone or the like. As the forehead pad 10 is "sandwiched" between the upper arm 3 and the patient's forehead, it is able to flex or crush (i.e. arrows PF in Fig. 4), while a pulling force (PF) is applied to the headgear 6 that is attached to the upper arm 3 as shown.

In prior art forehead pads, the flexing or crushing of the forehead pad 10 creates a kind of "sagging" of the main body 11 with a lateral sliding (LS ; arrows LS in Fig. 5) of its flexible peripheral wall that causes instabilities of use. In particular, the lateral sliding results in a mask 1 that is unstable during use, i.e while donned by the patient P, due to undesired lateral motions of the upper arm 3. The instability results from a rolling of the silicone pad 10 on the patient's forehead.

One goal of the present disclosure is to limit said lateral sliding as shown in Fig. 5 (see arrows LS), by providing an improved structure of the forehead pad 10 as shown in Fig. 2 & 3.

The forehead pad 10 according to the present disclosure further comprises a plurality of reinforcing members 13, also called "ribs" or similar, for instance 4 reinforcing members 13, arranged in the inner volume 12 of the hollow main body 11. The reinforcing members 13 have preferably an elongated shape and are axially-arranged along the axis XX of the main body 11.

Each reinforcing member 13 comprises a first edge 13.1, preferably a first longitudinal edge, secured to the inner wall 11.1 of the hollow main body 11, and a second edge 13.2, preferably a second longitudinal edge, secured to the other reinforcing members 13 in a common junction region 15 situated in the center region 12.1 of the inner volume 12 of the hollow main body 11, namely in the center area of its lumen as shown in Fig. 3.

The elongated reinforcing members 13 preferably have a blade shape or the like. The first longitudinal edge 13.1 of each elongated reinforcing member 13 is substantially parallel to its second longitudinal edge 13.2.

The 4 reinforcing members 13 are attached together along their second edges 13.2, in the junction region 15, so as to exhibit a substantially "X"- shape structure, when shown in cross-sectional view, as shown in Fig. 3. In other words, the reinforcing members 13 are substantially radially-arranged in the lumen of the hollow main body 11.

Preferably, the reinforcing members 13 have a curved shape, namely a curved blade shape, so as to exhibit a curved-arm "X"- shape structure, when shown in cross-sectional view, as shown in Fig. 3.

The reinforcing members 13 constitute a kind of inner ribs that maintain the overall structure of the hollow main body 11, especially while a pulling force (PF) is applied to the headgear 6, thereby limiting the sagging and the negative lateral sliding. Thanks to the reinforcing members 13 or ribs, the compression of the main body 11 of the forehead pad 10 is also more gradual, which further improves the comfort of use, i.e. the "feeling" of the patient and also helps the patient to reach a "perfect" level of adjustment or setting up of the mask.

The main body 11 can have a length L of for instance about 50 mm, whereas the elongated reinforcing members 13 arranged therein have preferably the same length.

As shown in Fig. 3, the main body 11 has a tubular shape comprising a quasi-cylindrical main portion 111 and a planar region 112 comprising the outer flat area 110.

Preferably, the hollow main body 11, the reinforcing members 13 and the fixation structure 14 are made one piece of a same resilient material, such as silicone or the like. The one-piece structure 11, 13, 14, namely the entire forehead pad 10, can be manufactured by an extrusion process or the like.

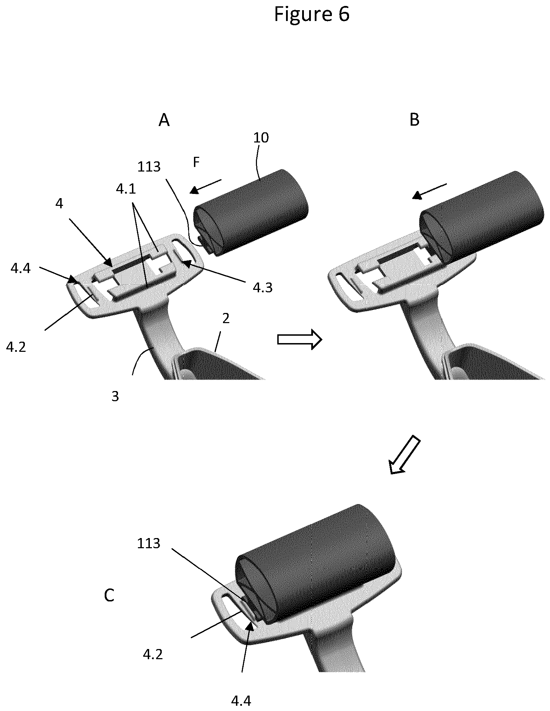

Figure 6 shows the coupling of the forehead pad 10 to the upper arm 3 of the respiratory mask 1 of Fig. 1.

In a first step A, the forehead pad 10 is provided in the direction of arrow F of Fig. 6, until the fixation structure 14 of the forehead pad 10 starts to cooperate with the forehead pad coupling structures 4 carried by the upper arm 3 of the mask 1.

In the present embodiment, the forehead pad coupling structures 4 comprise two guiding rails 4.1 that are parallel to each other. Those guiding rails 4.1 cooperates with the fixation structure 14 of the forehead pad 10, in particular with its base part 14.1 for guiding the insertion of the forehead pad 10 in said guiding rails 4.1 and thereafter holding it firmly, in a second step B.

The forehead pad coupling structures 4 further comprises an abutment 4.2 for stopping the insertion of the forehead pad 10 between the guiding rails 4.1, as shown in step C.

The abutment 4.2 is for instance a little wall or a similar little structure, that is arranged in the end region 4.4 of the two guiding rails 4.1, that is located opposite to the entry region 4.3 of the guiding rails 4.1, i.e. the side of the guiding rails 4.1 that receives the forehead pad 10 at the start of its insertion (steps A & B).

The abutment 4.2 stops the course of the forehead pad 10, at the end its insertion between the rails 4.1, namely as soon as the front surface 113 of the fixation structure 14 of forehead pad 10, namely the elongated base part 14.1, abuts against said abutment 4.2.

Generally speaking, the mask 1 of the present disclosure is light and comfortable to wear, and provides a good tightness (i.e. seal). It can be used in a method for treatment of a respiratory disorder or condition affecting infant, toddler, child or adult patients, such as Obstructive Sleep Apnea Syndrome (OSAS), Obesity Hypoventilation Syndrome (OHS), Neuromuscular disease (NMD or other respiratory troubles.

The scope of the present invention is defined by the following claims.

## Claims

1. Forehead pad (10) for a respiratory mask (1) comprising:
- a hollow main body (11) having an elongated tubular shape and comprising an inner volume (12), and
- a fixation structure (14) secured to an outer wall (11.2) of the hollow main body (11) and configured for being attachable to the respiratory mask (1),
wherein :
- the forehead pad further comprises at least 4 reinforcing members (13) arranged in the inner volume (12) of the hollow main body (11), each reinforcing member (13) comprising a first edge (13.1) secured to an inner wall (11.1) of the hollow main body (11) and a second edge (13.2) secured to at least one of the other reinforcing members (13), and
- at least the hollow main body (11) and the reinforcing members (13) are made of a resilient material,
**characterized in that** 4 reinforcing members (13) of said at least 4 reinforcing members (13) are attached together along their second edges (13.2) so as to form a substantially "X"- shape structure, in cross-sectional view.

2. Forehead pad according to Claim 1, **characterized in that** it comprises 4 reinforcing members (13).

3. Forehead pad according to Claim 1 or 2, **characterized in that** the reinforcing members (13) have an elongated shape, each elongated reinforcing member (13) comprising a first longitudinal edge (13.1) secured to the inner wall (11.1) of the hollow main body (11) and a second longitudinal edge (13.2) secured to at least one of the other elongated reinforcing members (13).

4. Forehead pad according to any one of the preceding Claims, **characterized in that** the second edges (13.2) of the reinforcing members (13) are secured together in a common junction region (15).

5. Forehead pad according to Claim 4, **characterized in that** the junction region (15) is located in a center region (12.1) of the inner volume (12) of the hollow main body (11).

6. Forehead pad according to Claim 3, **characterized in that** the elongated reinforcing members (13) are substantially radially-arranged (XX) in the inner volume (12) of the hollow main body (11).

7. Forehead pad according to any one of Claims 3 or 6, **characterized in that** the elongated reinforcing members (13) have a blade or plate shape, preferably the elongated reinforcing members (13) are curved.

8. Forehead pad according to any one of the preceding Claims, **characterized in that** the 4 reinforcing members (13) are attached together along their second edges (13.2) so as to form a substantially curved-arm "X"- shape structure.

9. Forehead pad according to Claim 1, **characterized in that** the fixation structure (14) comprises an elongated base part (14.1) and an elongated neck part (14.2) secured together, the neck part (14.2) linking the base part (14.1) to the outer wall (11.2) of the hollow main body (11).

10. Forehead pad according to Claim 9, **characterized in that** the base part (14.1) of the fixation structure (14) has a base width (W₁) that is larger than a neck width (W₂) of the neck part (14.2), i.e. W₁ > W₂, preferably the base part (14.1) forms with the neck part (14.2), a substantially "T"-shape structure, in cross-sectional view.

11. Forehead pad according to any one of Claims 9 or 10, **characterized in that** the base part (14.1) has a plate or blade shape.

12. Forehead pad according to any one of the preceding Claims, **characterized in that** the hollow main body (11), the reinforcing members (13) and the fixation structure (14) are made one piece of resilient material.

13. Forehead pad according to Claim 1 or 12, **characterized in that** the resilient material is chosen among silicone materials.

14. Respiratory mask (1), preferably a facial mask, comprising :
- a mask body (2) comprising an upper arm (3) projecting upwardly,
- forehead pad coupling structures (4) arranged at a free end (3.1) of the upper arm (3), and
- a forehead pad (10) according to any one of the preceding Claims secured to the forehead pad coupling structures (4).

15. Respiratory mask according to Claim 14, **characterized in that** the forehead pad coupling structures (4) comprise two guiding rails (4.1) that are parallel to each other, and an abutment (4.2).

## Patentansprüche

1. Stirnpolster (10) für eine Atemschutzmaske (1), umfassend:
einen hohlen Hauptkörper (11) mit einer länglichen röhrenförmigen Gestalt und einem Innenvolumen (12), und eine Befestigungsstruktur (14), die an einer Außenwand (11.2) des hohlen Hauptkörpers (11) befestigt und zur Anbringung an der Atemschutzmaske (1) konfiguriert ist, wobei: das Stirnpolster ferner mindestens 4 Verstärkungselemente (13) umfasst, die im Innenvolumen (12) des hohlen Hauptkörpers (11) angeordnet sind, wobei jedes Verstärkungselement (13) eine erste Kante (13.1), die an einer Innenwand (11.1) des hohlen Hauptkörpers (11) befestigt ist, und eine zweite Kante (13.2), die an mindestens einem der anderen Verstärkungselemente (13) befestigt ist, umfasst, und mindestens der hohle Hauptkörper (11) und die Verstärkungselemente (13) aus einem elastischen Material hergestellt sind, **dadurch gekennzeichnet, dass** 4 Verstärkungselemente (13) der besagten mindestens 4 Verstärkungselemente (13) entlang ihrer zweiten Kanten (13.2) so miteinander verbunden sind, dass sie im Querschnitt eine im Wesentlichen "X"-förmige Struktur bilden.

2. Stirnpolster nach Anspruch 1, **dadurch gekennzeichnet, dass** es 4 Verstärkungselemente (13) umfasst.

3. Stirnpolster nach Anspruch 1 oder 2, **dadurch gekennzeichnet, dass** die Verstärkungselemente (13) eine längliche Form aufweisen, wobei jedes längliche Verstärkungselement (13) eine erste Längskante (13.1), die an der Innenwand (11.1) des hohlen Hauptkörpers (11) befestigt ist, und eine zweite Längskante (13.2), die an mindestens einem der anderen länglichen Verstärkungselemente (13) befestigt ist, umfasst.

4. Stirnpolster nach einem der vorhergehenden Ansprüche, **dadurch gekennzeichnet, dass** die zweiten Kanten (13.2) der Verstärkungselemente (13) in einem gemeinsamen Verbindungsbereich (15) miteinander verbunden sind.

5. Stirnpolster nach Anspruch 4, **dadurch gekennzeichnet, dass** sich der Verbindungsbereich (15) in einem Mittenbereich (12.1) des Innenvolumens (12) des hohlen Hauptkörpers (11) befindet.

6. Stirnpolster nach Anspruch 3, **dadurch gekennzeichnet, dass** die länglichen Verstärkungselemente (13) im Wesentlichen radial (XX) im Innenvolumen (12) des hohlen Hauptkörpers (11) angeordnet sind.

7. Stirnpolster nach einem der Ansprüche 3 oder 6, **dadurch gekennzeichnet, dass** die länglichen Verstärkungselemente (13) eine Klingen- oder Plattenform aufweisen, vorzugsweise sind die länglichen Verstärkungselemente (13) gekrümmt.

8. Stirnpolster nach einem der vorhergehenden Ansprüche, **dadurch gekennzeichnet, dass** die 4 Verstärkungselemente (13) entlang ihrer zweiten Kanten (13.2) so miteinander verbunden sind, dass sie eine im Wesentlichen "X"-förmige Struktur mit gekrümmten Armen bilden.

9. Stirnpolster nach Anspruch 1, **dadurch gekennzeichnet, dass** die Befestigungsstruktur (14) einen länglichen Basisteil (14.1) und einen länglichen Halsteil (14.2) umfasst, die miteinander verbunden sind, wobei der Halsteil (14.2) den Basisteil (14.1) mit der Außenwand (11.2) des hohlen Hauptkörpers (11) verbindet.

10. Stirnpolster nach Anspruch 9, **dadurch gekennzeichnet, dass** der Basisteil (14.1) der Befestigungsstruktur (14) eine Basisbreite (W1) aufweist, die größer ist als eine Halsbreite (W2) des Halsteils (14.2), d. h. W• der Basisteil (14.1) mit dem Halsteil (14.2) im Querschnitt eine im Wesentlichen "T"-förmige Struktur.

11. Stirnpolster nach einem der Ansprüche 9 oder 10, **dadurch gekennzeichnet, dass** der Basisteil (14.1) eine Platten- oder Klingenform aufweist.

12. Stirnpolster nach einem der vorhergehenden Ansprüche, **dadurch gekennzeichnet, dass** der hohle Hauptkörper (11), die Verstärkungselemente (13) und die Befestigungsstruktur (14) einstückig aus elastischem Material hergestellt sind.

13. Stirnpolster nach Anspruch 1 oder 12, **dadurch gekennzeichnet, dass** das elastische Material aus Silikonmaterialien ausgewählt ist.

14. Atemschutzmaske (1), vorzugsweise eine Gesichtsmaske, umfassend:
- einen Maskenkörper (2), der einen sich nach oben erstreckenden oberen Arm (3) umfasst, Stirnpolster-Kopplungsstrukturen (4), die an einem freien Ende (3.1) des oberen Arms (3) angeordnet sind, und
- ein Stirnpolster (10) nach einem der vorhergehenden Ansprüche, das an den Stirnpolster-Kopplungsstrukturen (4) befestigt ist.

15. Atemschutzmaske nach Anspruch 14, **dadurch gekennzeichnet, dass** die Stirnpolster-Kopplungsstrukturen (4) zwei Führungsschienen (4.1), die parallel zueinander sind, und einen Anschlag (4.2) umfassen.

## Revendications

1. Tampon frontal (10) pour un masque respiratoire (1) comprenant : un corps principal creux (11) ayant une forme tubulaire allongée et comprenant un volume intérieur (12), et une structure de fixation (14) fixée à une paroi extérieure (11.2) du corps principal creux (11) et configurée pour être attachable au masque respiratoire (1), dans lequel : le tampon frontal comprend en outre au moins 4 éléments de renforcement (13) agencés dans le volume intérieur (12) du corps principal creux (11), chaque élément de renforcement (13) comprenant un premier bord (13.1) fixé à une paroi intérieure (11.1) du corps principal creux (11) et un second bord (13.2) fixé à au moins un des autres éléments de renforcement (13), et au moins le corps principal creux (11) et les éléments de renforcement (13) sont faits d'un matériau élastique, **caractérisé en ce que** 4 éléments de renforcement (13) desdits au moins 4 éléments de renforcement (13) sont attachés ensemble le long de leurs seconds bords (13.2) de manière à former une structure sensiblement en forme de « X », en coupe transversale.

2. Tampon frontal selon la revendication 1, **caractérisé en ce qu'**il comprend 4 éléments de renforcement (13).

3. Tampon frontal selon la revendication 1 ou 2, **caractérisé en ce que** les éléments de renforcement (13) ont une forme allongée, chaque élément de renforcement allongé (13) comprenant un premier bord longitudinal (13.1) fixé à la paroi intérieure (11.1) du corps principal creux (11) et un second bord longitudinal (13.2) fixé à au moins un des autres éléments de renforcement allongés (13).

4. Tampon frontal selon l'une quelconque des revendications précédentes, **caractérisé en ce que** les seconds bords (13.2) des éléments de renforcement (13) sont fixés ensemble dans une région de jonction commune (15).

5. Tampon frontal selon la revendication 4, **caractérisé en ce que** la région de jonction (15) est située dans une région centrale (12.1) du volume intérieur (12) du corps principal creux (11).

6. Tampon frontal selon la revendication 3, **caractérisé en ce que** les éléments de renforcement allongés (13) sont agencés sensiblement radialement (XX) dans le volume intérieur (12) du corps principal creux (11).

7. Tampon frontal selon l'une quelconque des revendications 3 ou 6, **caractérisé en ce que** les éléments de renforcement allongés (13) ont une forme de lame ou de plaque, de préférence les éléments de renforcement allongés (13) sont incurvés.

8. Tampon frontal selon l'une quelconque des revendications précédentes, **caractérisé en ce que** les 4 éléments de renforcement (13) sont attachés ensemble le long de leurs seconds bords (13.2) de manière à former une structure sensiblement en forme de « X » à bras incurvés.

9. Tampon frontal selon la revendication 1, **caractérisé en ce que** la structure de fixation (14) comprend une partie de base allongée (14.1) et une partie de col allongée (14.2) fixées ensemble, la partie de col (14.2) reliant la partie de base (14.1) à la paroi extérieure (11.2) du corps principal creux (11).

10. Tampon frontal selon la revendication 9, **caractérisé en ce que** la partie de base (14.1) de la structure de fixation (14) a une largeur de base (W1) qui est plus grande qu'une largeur de col (W2) de la partie de col (14. de préférence la partie de base (14.1) forme avec la partie de col (14.2) une structure sensiblement en forme de « T », en coupe transversale.

11. Tampon frontal selon l'une quelconque des revendications 9 ou 10, **caractérisé en ce que** la partie de base (14.1) a une forme de plaque ou de lame.

12. Tampon frontal selon l'une quelconque des revendications précédentes, **caractérisé en ce que** le corps principal creux (11), les éléments de renforcement (13) et la structure de fixation (14) sont faits d'une seule pièce de matériau élastique.

13. Tampon frontal selon la revendication 1 ou 12, **caractérisé en ce que** le matériau élastique est choisi parmi des matériaux de silicone.

14. Masque respiratoire (1), de préférence un masque facial, comprenant :
- un corps de masque (2) comprenant un bras supérieur (3) se projetant vers le haut, des structures d'accouplement de tampon frontal (4) agencées à une extrémité libre (3.1) du bras supérieur (3), et
- un tampon frontal (10) selon l'une quelconque des revendications précédentes fixé aux structures d'accouplement de tampon frontal (4).

15. Masque respiratoire selon la revendication 14, **caractérisé en ce que** les structures d'accouplement de tampon frontal (4) comprennent deux rails de guidage (4.1) qui sont parallèles l'un à l'autre, et une butée (4.2).
